# EUROPÄISCHE PATENTSCHRIFT

(11) **EP 1 681 024 B1**
(45) Veröffentlichungstag und Bekanntmachung des Hinweises auf die Patenterteilung: **25.06.2008**
(21) Anmeldenummer: 06005740.3
(22) Anmeldetag: 20.12.2002
(51) Int. Cl.: A61B 17/86, A61B 17/70

(54) **Knochenverankerungselement**
Bone anchor element
Élément de fixation osseuse

(30) Priorität: 02.10.2002 DE 10246177
(43) Veröffentlichungstag der Anmeldung: 19.07.2006
(62) Teilanmeldung aus: 02795258.9
(73) Patentinhaber: BIEDERMANN MOTECH GmbH, 78054 VS-Schwenningen (DE)
(72) Erfinder: Biedermann, Lutz, 78048 VS-Villingen (DE); Harms, Jürgen, 76227 Karlsruhe (DE); Rapp, Helmar, 78652 Deisslingen (DE)
(74) Vertreter: Hofer, Dorothea

(56) Entgegenhaltungen:
- EP-A- 0 938 872
- WO-A-01/12088
- DE-A- 10 055 891
- DE-U- 29 903 855
- FR-A- 2 820 630
- US-A- 5 658 285

## Beschreibung

Die Erfindung betrifft ein Verankerungselement mit einer einen Schaft mit einem Knochengewindeabschnitt und einen Kopf aufweisenden Schraube und einem Aufnahmeteil zum Verbinden der Schraube mit einem Stab nach dem Oberbegriff des Patentanspruches 1. Ein solches Verankerungselement wird insbesondere in der Wirbelsäulenchirurgie, aber auch in der Unfallchirurgie eingesetzt.

Ein Verankerungselement ist zum Beispiel aus der DE 43 07 576 C1 bekannt.

Eine bekannte Behandlungsmethode zum Behandeln von Knochendefekten, insbesondere von osteoporotischen Frakturen, beinhaltet das Einspritzen von Knochenzement und/oder von medikamentösen Wirkstoffen, insbesondere von wachstumsfördernden Stoffen, in den Knochen. Insbesondere im Bereich der Wirbelsäule erfordert dies eine genaue Positionierung des einzuspritzenden Materials im Wirbel. Ferner ist es in den vielen Fällen erforderlich, die defekten Wirbel zusätzlich zu stabilisieren bzw. relativ zueinander zu fixieren.

Die US 5,658,285 offenbart eine Schraubenvorrichtung für ein Knochengelenk, speziell zum Stabilisieren von mindestens zwei Wirbeln, die einen einteiligen rohrförmigen Körper mit einem Gewinde auf seiner Außenseite beinhaltet. Der rohrförmige Körper ist auf seiner Innenseite mit länglichen Rillen versehen, sodass Öffnungen in dem Gewindegrund gebildet sind, die die Außenseite des Körpers mit dem Inneren des Körpers verbinden. Es ist ein Verschlusselement vorgesehen, das auf das offene Ende des rohrförmigen Körpers aufgeschraubt wird.

Der Oberbegriff des Anspruchs 1 basiert auf diesem Verankerungselement.

Aus der DE 100 55 891 A1 ist eine Knochenschraube mit einem rohrförmigen, ein Knochengewinde aufweisenden Gewindeabschnitt mit einer Mehrzahl von in der Wandung des Gewindeabschnitts vorgesehenen Öffnungen bekannt.

Aufgabe der Erfindung ist es, ein Verankerungselement der eingangs beschriebenen Art so zu verbessern, daß es insbesondere bei der Behandlung von osteoporotischen Frakturen einsetzbar ist.

Diese Aufgabe wird durch das in Patentanspruch 1 gekennzeichnete Verankerungselement gelöst. Dadurch wird es möglich, daß die Knochenschraube mit dem umgebenden Knochenmaterial fusionieren kann, sowie gleichzeitig Knochenabschnitte oder Wirbel relativ zueinander positioniert und fixiert werden können. Ferner kann ein in den Knochen einzubringendes Material präzise an die erforderliche Stelle eingebracht werden.

Weiterbildungen der Erfindung sind in den Unteransprüchen gekennzeichnet.

Weitere Merkmale und Zweckmäßigkeiten der Erfindung ergeben sich aus der Beschreibung von Beispielen, die keine Ausführungsform der Erfindung sind, anhand der Figuren.

Von den Figuren zeigen:
- Fig. 1: eine Explosionsdarstellung eines ersten Verankerungselements;
- Fig. 2: eine Seitenansicht des Verankerungselements von Fig. 1 in geschnittener Darstellung;
- Fig.3: einen Ausschnitt einer Abwandlung des Verankerungselements nach Fig. 1 in geschnittener Darstellung;
- Fig. 4: einen Ausschnitt einer weiteren Abwandlung des Verankerungselements in geschnittener Darstellung;
- Fig. 5: eine Explosionsdarstellung eines zweiten Verankerungselements;
- Fig. 6: eine Seitenansicht des zweiten Verankerungselements in geschnittener Darstellung.
- Fig. 7a): ein weiteres Verankerungselement;
- Fig. 7b): ein weiteres Verankerungselement;
- Fig. 8: ein weiteres Verankerungselement;
- Fig. 9: eine Abwandlung;
- Fig. 10: eine vergrößerte teilgeschnittene Darstellung der Abwandlung von Fig. 9;
- Fig. 11: eine weitere Abwandlung in vergrößerter teilgeschnittener Darstellung; und
- Fig. 12: ein weitere Abwandlung.

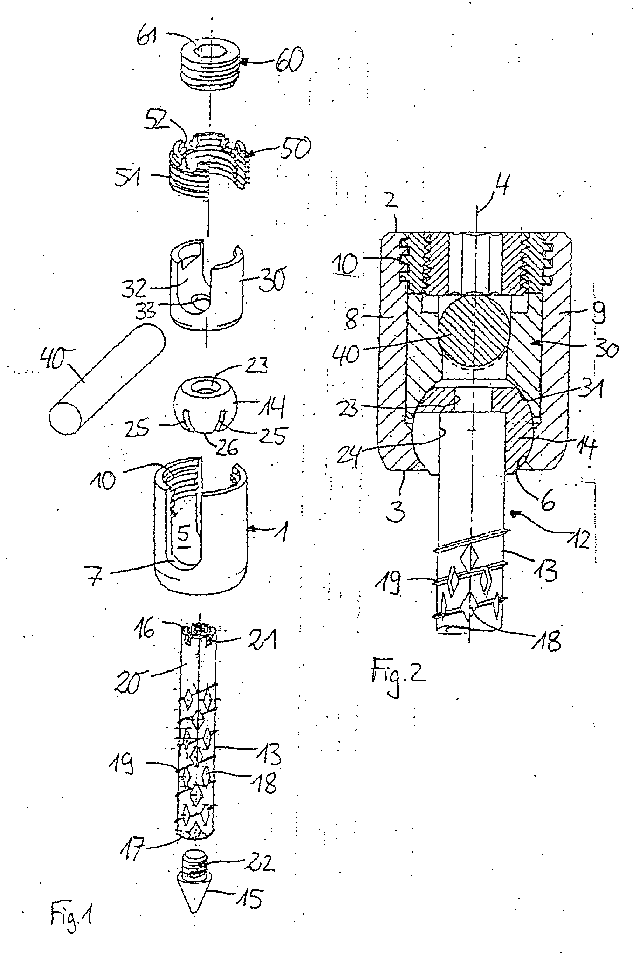

Bei den in dem Figuren 1 und 2 gezeigten ersten Beispiel weist das Verankerungselement ein zylindrisch ausgebildetes Aufnahmeteil 1 mit einem ersten Ende 2 und einem gegenüberliegenden zweiten Ende 3 auf. Die beiden Enden erstrecken sich senkrecht zu einer Symmetrie- bzw. Längsachse 4. Koaxial zu der Längsachse 4 ist eine sich von dem ersten Ende 2 aus erstreckende erste koaxiale Bohrung 5 vorgesehen, die sich bis zu einem vorbestimmten Abstand von dem zweiten Ende 3 hin erstreckt. An dem zweiten Ende 3 ist eine zweite Bohrung 6 vorgesehen, deren Durchmesser kleiner als der Durchmesser der ersten Bohrung ist. In dem gezeigten Beispiel ist die zweite Bohrung als eine Öffnung ausgebildet, deren Rand als hohlkugelsegmentförmiger Abschnitt geformt ist, dessen Mittelpunkt zum ersten Ende 2 hin gerichtet ist.

Das Aufnahmeteil 1 weist ausgehend von dem ersten Ende 2 eine sich senkrecht zu Längsachse 3 erstreckende U-förmige Ausnehmung 7 auf mit zwei zum ersten Ende 2 hin endenden freien Schenkeln 8, 9. Angrenzend an das erste Ende 2 weisen die Schenkel einen Innengewinde 10 auf. Das Innengewinde ist zum Beispiel als Flachgewinde mit Gewindeflanken, die sich jeweils unter einem Winkel von 90° zur Symmetrieachse 4 erstrecken, ausgebildet. Der Grund der U-förmigen Ausnehmung erstreckt sich bis zu einem vorgegebenen Abstand von dem zweiten Ende 3 hin.

Die mit dem Aufnahmeteil 1 zusammenwirkende Schraube 12 weist einen Schraubenschaft 13 mit einem Gewindeabschnitt und einen in der in Fig. 2 gezeigten montierten Darstellung mit dem Schraubenschaft verbundenen kugelsegmentförmigen Kopf 14 sowie eine Spitze 15 auf.

Der Schraubenschaft 13 ist rohrförmig ausgebildet und weist ein erstes, dem Kopf 14 zugewandtes Ende 16 und ein diesem gegenüberliegendes Ende 17 auf. In seiner Wandung weist der rohrförmige Schraubenschaft 13 eine Vielzahl von Ausnehmungen 18 auf, die in dem gezeigten Beispiel rautenförmig ausgebildet sind. Dabei erfolgt die Ausrichtung der Rauten derart, daß jeweils eine Symmetrieachse sich parallel zur Symmetrieachse des Rohres erstreckt. In axialer Richtung sind die Ausnehmungen 18 gegeneinander versetzt derart angeordnet, daß jeweils eine Öffnung zwischen den Öffnungen der vorhergehenden in Umfangsrichtung angeordneten Reihe von Öffnungen ist. Auf der Außenwandung ist in einem Bereich, der sich vom zweiten Ende 17 des Schraubenschafts 13 bis wenigstens zu einem vorbestimmten Abstand von dem ersten Ende 16 hin erstreckt, ein sogenanntes Knochengewinde 19 vorgesehen, welches in seiner Form den Knochengewinden üblicher Knochenschrauben entspricht. Der rohrförmige Schraubenschaft 13 weist ferner in dem gezeigten Beispiel angrenzend an das erste Ende 16 einen Abschnitt 20 auf, in dem kein Knochengewinde 19 gebildet ist und dessen Oberfläche im wesentlichen glatt ausgebildet ist. Ferner sind zum Einschrauben stirnseitig an dem ersten Ende 16 Schlitze'21 für einen Schraubendreher vorgesehen.

Die Spitze 15 umfaßt den eigentlichen Spitzenteil und einen Schaft 22, der in dem gezeigten Beispiel ein metrisches Außengewinde aufweist. An seiner Innenwandung angrenzend an das zweite Ende 17 weist der rohrförmige Schraubenschaft 13 ein Abschnitt mit einem entsprechenden metrischen Innengewinde auf, und die Spitze ist in montiertem Zustand durch Einschrauben fest mit dem rohrförmigen Schraubenschaft verbunden.

Wie am besten aus Fig. 1 zu ersehen ist, ist der Kopf 14 als eine an ihrem dem ersten Ende 2 des Aufnahmteils 1 zuzuwendenden Ende abgeflachte Kugel ausgebildet und weist eine erste zu der Längsachse 4 koaxiale durchgehende Bohrung 23 mit einem Durchmesser auf, der kleiner ist, als der Durchmesser des rohrförmigen Schraubenschafts 13. Es ist ferner eine koaxiale zweite Bohrung 24 vorgesehen, die sich von dem in montiertem Zustand dem zweiten Ende 3 des Aufnahmeteils zugewandten Ende des Kopfs 14 bis zu einem vorbestimmten Abstand in den Kopf hinein erstreckt und deren Durchmesser gleich dem Außendurchmesser des rohrförmigen Schraubenschafts 13 in dem Abschnitt 20 ist, so daß der Abschnitt 20 des Schraubenschaftes in die Bohrung 24 reibschlüssig einschiebbar ist. Wie aus Fig. 1 ersichtlich ist, ist der so geformte hohlkugelsegmentförmige Kopf 14 auf seiner dem abgeflachten Ende gegenüberliegenden Seite mit in Umfangsrichtung einen Abstand zueinander aufweisenden und sich parallel zur Längsachse 4 erstreckenden und bis zu dem der abgeflachten Seite gegenüberliegenden Ende reichenden Ausschnitten 25 versehen. Dadurch wird erreicht, daß der dem ersten Ende 2 des Aufnahmeteils in eingesetztem Zustand abgewandte Rand 26 zum Einführen des Schraubenschaftes 13 federnd nach außen nachgebbar ausgebildet ist.

Es ist ferner ein Druckelement 30 vorgesehen, welches zylindrisch ausgebildet ist und einen Außendurchmesser aufweist, der gerade so groß ist, daß das Druckelement in die erste Bohrung 5 einführbar und in dieser in Axialrichtung hin- und herbewegbar ist. Auf seiner dem zweiten Ende 3 zugewandten Unterseite weist das Druckelement 30 einen zur Längsachse 4 symmetrisch ausgebildeten hohlkugelsegmentförmigen Abschnitt 31 auf, dessen Radius dem Radius des Kopfes 14 entspricht. Das Druckelement weist ferner eine sich quer zur Längsachse 4 erstreckende U-förmige Ausnehmung 32 auf, deren freie Schenkel sich zum ersten Ende 2 hin erstrecken und die einen Kanal für einen aufzunehmender Stab 40 bildet. Die Tiefe der U-förmigen Ausnehmung ist größer, als der Durchmesser des einzusetzenden Stabes 40, so daß in montiertem Zustand die Schenkel des Drukkelements 30 über den eingelegten Stab 40 hinaus hervorstehen. Am Grund der U-förmigen Ausnehmung 32 schließt sich eine koaxiale Bohrung 33 an, die zum Eingreifen mit einem Schraubwerkzeug dient.

Zum Fixieren der Stellung des Kopfes 14 mit dem eingesetzten Schraubenschaft 13 relativ zu dem Aufnahmeteil 1 ist eine zwischen die Schenkel 8, 9 des Aufnahmeteils einschraubbare Mutter 50 mit einem Außengewinde 51 vorgesehen, welches mit dem Innengewinde 11 der Schenkel zusammenwirkt. Die Mutter weist an ihrem einen Ende Schlitze 52 zum Ineingriffbringen mit einem Schraubwerkzeug auf.

Ferner ist eine Innenschraube 60 zum Einschrauben in die Mutter 50 vorgesehen, die ein Außengewinde aufweist, welches mit dem Innengewinde der Mutter 50 zusammenwirkt. Die Innenschraube 60 weist eine Ausnehmung 61 zum Ineingriffbringen mit einem Schraubwerkzeug auf.

Im Betrieb wird zunächst die Schraube 12 in den Knochen bzw. dem Wirbel eingeschraubt. Dann wird über eine Spritze Knochenzement oder ein anderes Füllmaterial und/oder ein Wirkstoff in den rohrförmigen Schaft eingespritzt. Anschließend wird das Aufnahmeteil 1 mit der zweiten Bohrung 6 auf den Schaft 13 aufgesetzt und der Kopf 14 von dem ersten Ende 2 her auf den Schaft 13 geführt, so daß der Schaft 13 mit seinem knochengewindefreien Abschnitt 20 in die Bohrung 24 eingeführt wird und der Kopf den Schaft in der in Fig. 2 gezeigten Weise einschließt. Der Kopf 14 und der Schaft 13 sind reibschlüssig miteinander verbunden. Anschließend wird das Druckelement 30 eingesetzt und durch Einschrauben der Mutter 50 so auf den mit den Schlitzen 25 versehenen Kopf 14 gedrückt, daß dieser einerseits mit dem Schaft 13 bewegungsfest verbunden bzw. verklemmt wird und gleichzeitig gegen den hohlkugelsegmentförmigen Abschnitt in dem Aufnahmeteil gedrückt und somit in seiner Drehstellung arretiert wird. Der Stab 40 ist noch frei verschiebbar. Dieser wird anschließend durch Einschrauben der Innenschraube 60 fixiert. Mit dem Verankerungselement ist somit eine Behandlung eines defekten Knochens mit einem Wirkstoff und/oder eine Stabilisierung durch Fusion mit umgebendem Knochenmaterial und gleichzeitig eine Positionierung und Fixierung von Knochenstücken oder von Wirbeln über den Stab möglich.

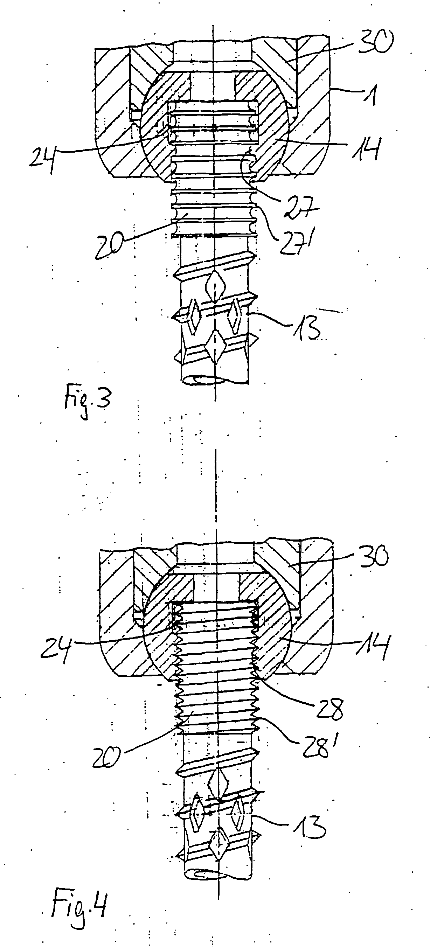

Die in der Figur 3 gezeigte Abwandlung unterscheidet sich von dem in den Figuren 1 und 2 gezeigten Beispiel dadurch, daß die Innenwand der Bohrung 24 des Kopfes 14 mit in Umfangsrichtung vorgesehenen Wellen 27 ausgebildet ist und der Abschnitt 20 des Schaftes 13 mit entsprechenden Wellen 27' versehen ist.

Bei der in Fig. 4 gezeigten Abwandlung weisen der Kopf 14 und der Schaft 13 anstelle der Wellen zueinander passende Gewinde 28, 28' auf, so daß der Schaft in den Kopf einschraubbar ist.

Der Schraubenschaft 13 kann auch andere Mittel aufweisen über die er in den Knochen einschraubbar ist. Beispielsweise kann der Schraubenschaft 13 ein Innengewinde auch angrenzend an sein erstes Ende oder ein sich über die gesamte axiale Länge erstreckendes Innengewinde aufweisen. In diesem Fall kann der Schaft über einen einzuschraubende Kopf oder ein anderes Hilfsinstrument, welches nach dem Einschrauben wieder entfernt wird, eingeschraubt werden. Alternativ ist es möglich, daß der Schraubenschaft 13 angrenzend an sein erstes Ende innen sechskantförmig zum Eingreifen mit einem Inbusschlüssel ausgebildet ist.

Es ist auch möglich, daß der Schraubenschaft 13 vor dem Einschrauben mit Knochenmaterial gefüllt wird, welches dann mit dem die Schraube umgebenden Knochenmaterial nach dem Einschrauben verwächst.

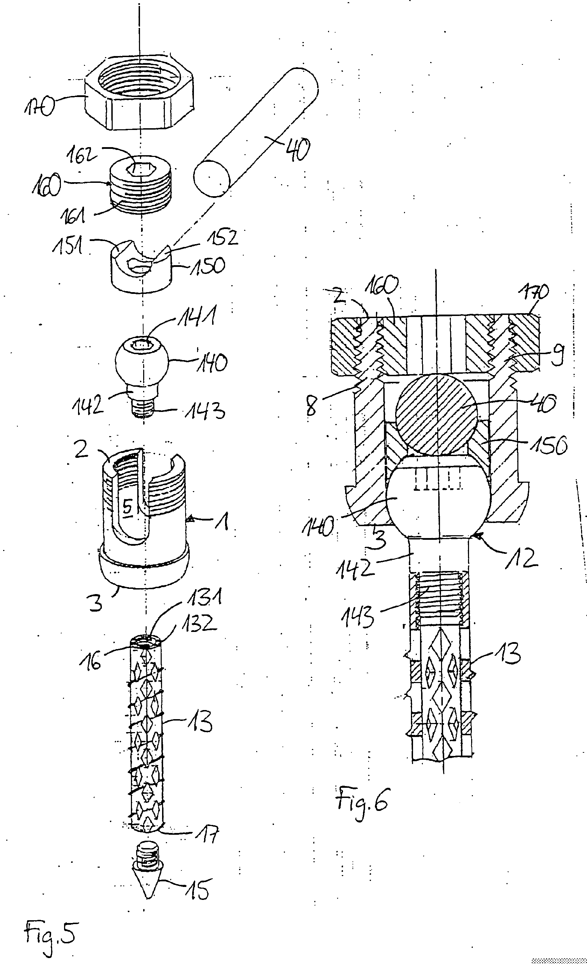

Das in den Figuren 5 und 6 dargestellte Beispiel unterscheidet sich von dem in den Figuren 1 und 2 gezeigten Beispiel im wesentlichen durch die Ausbildung des Schraubenkopfs 140 und seiner Verbindung mit dem Schraubenschaft 13.

Der Schraubenkopf 140 ist kugelsegmentförmig ausgebildet mit einem Kugelradius, der im wesentlichen gleich dem Radius des hohlkugelsegmentförmigen Abschnitts des Aufnahmeteils ist. Der Kopf weist ferner an seinem zu dem ersten Ende 2 des Aufnahmeteils 1 hin zu richtenden abgeflachten Ende eine Ausnehmung 141 zum Ineingriffbringen mit einem Schraubendreher auf. An seinem gegenüberliegenden Ende besitzt der Schraubenkopf 140 einen zylindrischen Hals 142 mit einem Außendurchmesser, der dem Außendurchmesser des rohrförmigen Schraubenschafts 13 entspricht. Von dem Hals erstreckt sich ein Ansatz 143 mit einem Außengewinde mit dem der Schraubenkopf in den rohrförmigen Schraubenschaft 13 einschraubbar ist, welcher hierzu angrenzend an sein erstes Ende 16 an der Innenwandung ein Innengewinde 131 aufweist. Somit erfolgt im Gegensatz zu der ersten Ausführungsform die Verbindung des Kopfes mit dem Schraubenschaft derart, daß der Kopf in den Schaft eingreift, während bei dem ersten Beispiel der Kopf den Schaft umgreift.

Der Schraubenkopf 140 in dieser Beispiel kann zweckmäßigerweise eine durchgehende koaxiale Bohrung aufweisen, die in den Figuren nicht dargestellt ist, und welche als Kanal zu Einführen von Wirkstoffen dient.

Angrenzend an das zweite Ende 17 des rohrförmigen Schraubenschaftes 13 ist wie bei dem ersten Beispiel an der Innenwandung ebenfalls ein Innengewinde zum Einschrauben der Spitze 15 vorgesehen. Wie auch schon bei der ersten Ausführungsform kann das Innengewinde über die gesamte Länge des rohrförmigen Gewindeschafts ausgebildet sein, was herstellungstechnisch günstig ist und außerdem das Kürzen des rohrförmigen Schraubenschafts auf eine gewünschte Länge erlaubt. An dem ersten Ende des Schraubenschaftes 13 können Schlitze 132 zum Eingreifen mit einem Schraubendreher vorgesehen sein.

In dem dargestellten Beispiel ist eine Variante der bei dem ersten Beispiel gezeigten Kopf- und Stabfixierung dargestellt. Das Druckelement 150 weist im Gegensatz zu dem Druckelement 50 des ersten Beispiels nur kurze Schenkel 151, 152 auf, die nicht seitlich über den eingelegten Stab 40 überstehen. Ansonsten weist das Druckelement wie bei der ersten Beispiel eine sphärische Ansenkung 153 an seiner dem Kopf zugewandten Seite und eine koaxiale Bohrung 154 auf.

Zum Fixieren von Kopf und Stab ist eine Innenschraube 160 mit einem Außengewinde 161, welches dem Innengewinde der Schenkel des Aufnahmeteils entspricht, und mit einer Ausnehmung zum Ineingriffbringen mit einem Schraubendreher vorgesehen. Zur Sicherung der Fixierung ist eine auf das Aufnahmeteil 1 aufschraubbare Sicherungsmutter 170 vorgesehen.

Im Betrieb wird zuerst die Spitze auf den Schraubenschaft 13 aufgeschraubt. Anschließend wird, falls erforderlich, Knochenmaterial in den rohrförmigen Schraubenschaft eingefüllt und der Kopf 140 aufgeschraubt. Dann wird die aus miteinander verschraubten Schaft 13, Spitze 15 und Kopf 140 bestehende Schraube wie eine bekannte Polyaxialschraube in das Aufnahmeteil 1 eingeführt und in den Knochen eingeschraubt. Falls ein kanulierter Kopf 140 verwendet wird, kann ein Wirkstoff oder ein Füllmaterial eingespritzt werden. Schließlich wird das Druckelement eingesetzt und das Aufnahmeteil mit dem Stab durch Einschrauben der Innenschraube 160 und der Sicherungsmutter 170 fest verbunden und somit die Winkelstellung des Kopfs in dem Aufnahmeteil fixiert.

Alternativ kann auch, wenn der Schraubenschaft die Schlitze 132 zum Eingreifen mit einem Schraubendreher aufweist, zuerst der Schraubenschaft 13 mit aufgeschraubter Spitze 15 ohne den Kopf 140 eingeschraubt werden. Anschließend kann der Wirkstoff eingefüllt werden und das Aufnahmeteil aufgesetzt und der Schraubenkopf aufgeschraubt werden. Die Verbindung mit dem Stab erfolgt dann wie zuvor beschrieben.

Abwandlungen der beschriebenen Beispiel sind möglich. Zum einen ist die Kopf- und Stabfixierung nicht auf die beschriebenen Varianten beschränkt. Es kann bei dem ersten Beispiel auch die Kopf- und Stabfixierung des zweiten Beispiels verwendet werden und umgekehrt. Ferner können auch andere Ausbildungen, wie z.B. das Vorsehen von lediglich einer auf den Stab einwirkenden Innenschraube vorgesehen sein.

Anstelle der rautenförmigen Öffnungen im Schraubenschaft können auch kreisförmige, ovale oder andere, eine beliebige Form aufweisende Öffnungen vorgesehen sein. Die Öffnungen können sich ferner über die gesamte axiale Länge des Schraubenschaftes erstrecken.

Der Kopf 14 des ersten Beispiels kann an einer Stelle durchgehend in axialer Richtung geschlitzt sein. Durch die somit erzeugte Elastizität ist der Kopf etwas zusammendrückbar, so daß er von dem zweiten Ende 3 des Aufnahmeteils her einführbar ist.

Die Spitze 15 kann selbstschneidend ausgebildet sein. Ferner kann die Spitze einen koaxial verlaufenden durchgehenden Kanal zum Hindurchleiten von Wirkstoffen haben.

Der rohrförmige Schraubenschaft 13 kann eine für die jeweilige Anwendung geeignete Länge aufweisen, die gegebenenfalls durch Abschneiden eines Rohrstücks mit gewünschter Länge von einem längeren Rohrstück erzeugt wird, sowie einen der Anwendung entsprechenden Durchmesser. Insbesondere kann die Schraube auch als Pedikelschraube ausgebildet sein.

Das Verankerungselement kann zur Stabilisierung der Wirbelsäule oder von Knochen allgemein über den Stab mit bekannten Verankerungselementen kombiniert werden.

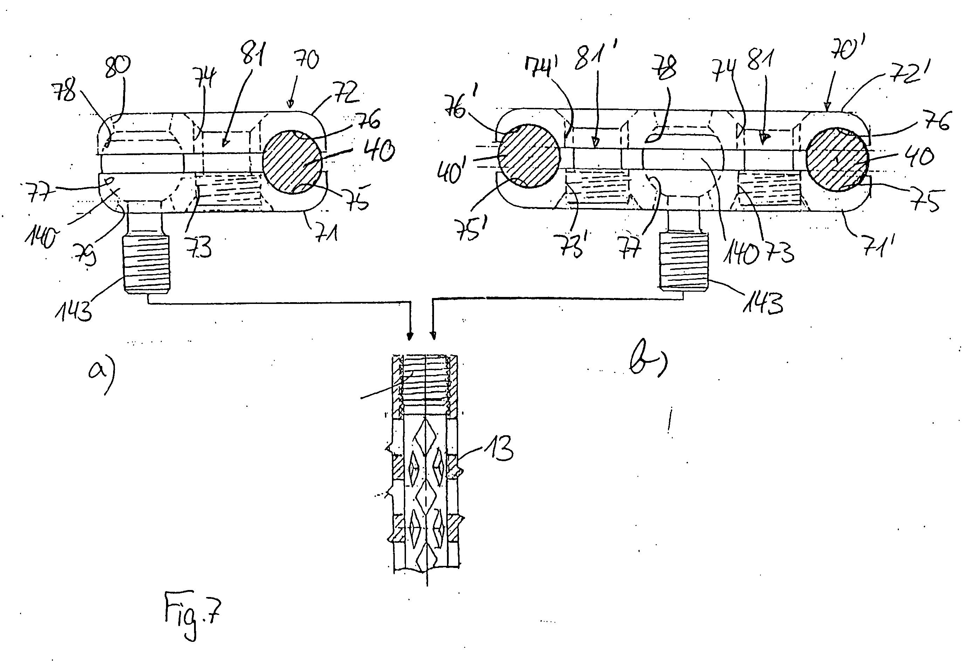

In dem in den Figuren 7a) und 7b) gezeigten Beispiel erfolgt die polyaxiale Verbindung mit dem Stab 40 nicht in Richtung der Schraubenachse, wie bei den vorhergehenden Beispielen, sondern seitlich zur Schraubenachse versetzt.

Das Verankerungselement gemäß Fig. 7a) umfaßt ein aus dem rohrförmigen Schraubenschaft 13, einer Spitze und dem kugelsegmentförmigen Kopf 140 bestehendes Schraubenelement sowie eine dem Kopf 140 aufnehmende zweiteilige Fassung 70 mit einem dem Schraubenschaft zugewandten Unterteil 71 und einem dem Schraubenschaft abgewandten Oberteil 72, die zusammen den Stab 40 umfassen. Das Unterteil 71 und das Oberteil 72 sind identisch ausgebildet und spiegelbildlich zueinander angeordnet. Sie weisen je eine zentrale Bohrung 73, 74 auf, die mit einem Innengewinde versehen ist und auf der dem jeweils anderen Teil 71, 72 abgewandten Oberfläche eine Senkbohrung aufweist. Seitlich der Bohrung 73, 74 ist in einem Abstand von dieser eine zu dem jeweils anderen Teil 71, 72 hin zylindersegmentförmig ausgebildete Ausnehmung 75, 76 zum Halten des Stabs 40 vorgesehen. Auf der anderen Seite der Bohrung 73, 74 weisen das Unterteil 71 und das Oberteil 72 auf der dem jeweils anderen Teil zugewandten Seite eine kugelsegmentförmige Ausnehmung 77, 78 zum Halten des Schraubenkopfs 40 auf. Auf der dem anderen Teil 71, 72 abgewandten Oberfläche schließt sich koaxial zu der Ausnehmung 77, 78 eine nach außen zunehmende Ausnehmung 79, 80 an.

Unterteil 71 und Oberteil 72 der Fassung sind durch eine Schraube 81 miteinander verbunden, die in das Innengewinde des Oberteils einführbar ist, und in das Innengewinde des Unterteils einschraubbar ist. In ihrem durch das Oberteil 72 geführten Teil hat die Schraube 81 einen Durchmesser, der kleiner ist, als der Durchmesser des Innengewindes des Oberteils und weist in ihrem durch das Unterteil geführten Teil ein mit dem Innengewinde des Unterteils zusammenwirkendes Außengewinde auf. Die zylindersegmentförmigen Ausnehmungen 75, 76 und die kugelsegmentförmigen Ausnehmungen 77, 78 sind so dimensioniert und so zueinander angeordnet, daß in dem Zustand in dem der Stab 40 und der Kopf 140 gehalten sind, das Unterteil 71 und das Oberteil 72 parallel zueinander ausgerichtet sind und einen Abstand voneinander besitzen.

Im Betrieb wird erst das Schraubenelement zusammengesetzt indem Spitze und Kopf 140 an den Schaft geschraubt werden. Das Oberteil und das Unterteil der Fassung sind durch Losedrehen der Schraube 81 um 90 Grad gegeneinander verdreht, so daß das Schraubenelement in das Unterteil einführbar ist. Das Schraubenelement wird eingeführt, bis sein Kopf 140 in der kugelsegmentförmigen Ausnehmung 77 des Unterteils 71 anliegt. Anschließend wird es in den Knochen eingeschraubt. Dann wird der Stab 40 aufgenommen und das Oberteil 72 um 90 Grad zum Fassen des Stabs gedreht. Nach erfolgter Justierung der Winkelstellung des Schraubenkopfs 140 in der Fassung und der Position des Stabs erfolgt ein Fixieren durch Festziehen der Schraube 81.

Das Implantat ist insbesondere für die Fixierung von Frakturen am Becken und an Röhrenknochen geeignet.

Das in Fig. 7b) dargestellte Beispiel unterscheidet sich von dem in Fig. 7a) dargestellten Beispiel dadurch, daß die Fassung 70' zum Erfassen von zwei Stäben 40, 40' ein in sich symmetrisch ausgebildetes Unterteil 71' und Oberteil 72' aufweist. Das Unterteil 71' und das Oberteil 72' sind dabei symmetrisch zu einer durch die Mittellinie der Stäbe 40, 40' und den Mittelpunkt des kugelsegmentförmigen Kopfs 140 der Schraube definierten Ebene ausgebildet und weisen jeweils zwei Bohrungen 73, 73' bzw. 74, 74', zwei zylindersegmentförmige Ausnehmungen 75, 75' bzw. 76, 76' auf. Zum Fixieren sind zwei Fixierschrauben 81, 81' vorgesehen. Der Betrieb ist analog zu dem vorherbeschriebenen Beispiel, nur daß zwei Stäbe zu fixieren sind.

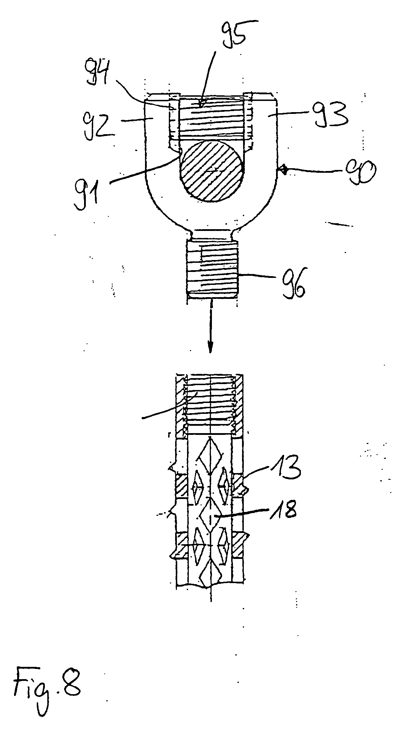

Bei dem in Fig. 8 gezeigten Beispiel besteht das Verankerungselement aus einem Schraubenelement, das durch den rohrförmigen Schraubenschaft 13 mit einer mit diesem verbundenen Spitze gebildet ist, und aus einem mit dem Schraubenelement monoaxial verbindbaren Aufnahmeteil 90 zum Aufnehmen eines Stabs 40. Das Aufnahmeteil 90 ist im wesentlichen zylindrisch ausgebildet und weist eine Ausnehmung 91 mit U-förmigem Querschnitt auf, die gerade so groß bemessen ist, daß der Stab 40 einlegbar ist und in den Grund der Ausnehmung passt. Durch die U-förmige Ausnehmung 91 sind zwei freie Schenkel 92, 93 gebildet. Angrenzend an das freie Ende weisen die Schenkel 92, 93 ein Innengewinde 94 auf, welches mit einem entsprechenden Außengewinde einer zwischen die Schenkel einzuschraubenden Innenschraube 95 zum Fixieren des Stabs 40 zusammenwirkt. An seinem dem freien Ende abgewandten Ende weist das Aufnahmeteil 90 einen Gewindeschaft 96 zum Einschrauben in den rohrförmigen Schaft 13 auf.

Im Betrieb wird das Verankerungselement vorzugsweise zuerst vollständig zusammengesetzt, wobei der rohrförmige Schaft wenn es erforderlich ist, mit Wirkstoffen oder Knochenmaterial gefüllt ist. Anschließend wird das Verankerungselement wie eine bekannte Monoaxialschraube in den Knochen eingeschraubt. Dann erfolgt die Verbindung über den Stab zu einem oder mehreren anderen Verankerungselementen. In korrekter Position wird anschließend der Stab über die Innenschraube fixiert.

Bei den in den Figuren 1 bis 8 gezeigten Beispielen sind die Ausnehmungen 18 teilweise so angeordnet, daß sie die Wendelspitze des Knochengewindes durchbrechen. Dadurch werden Zähne oder scharfe Kanten am Knochengewinde gebildet, die beim Einschrauben in den Knochen einen Fräseffekt haben. Für bestimmte Anwendungen ist jedoch ein glattes Einschrauben erwünscht oder erforderlich.

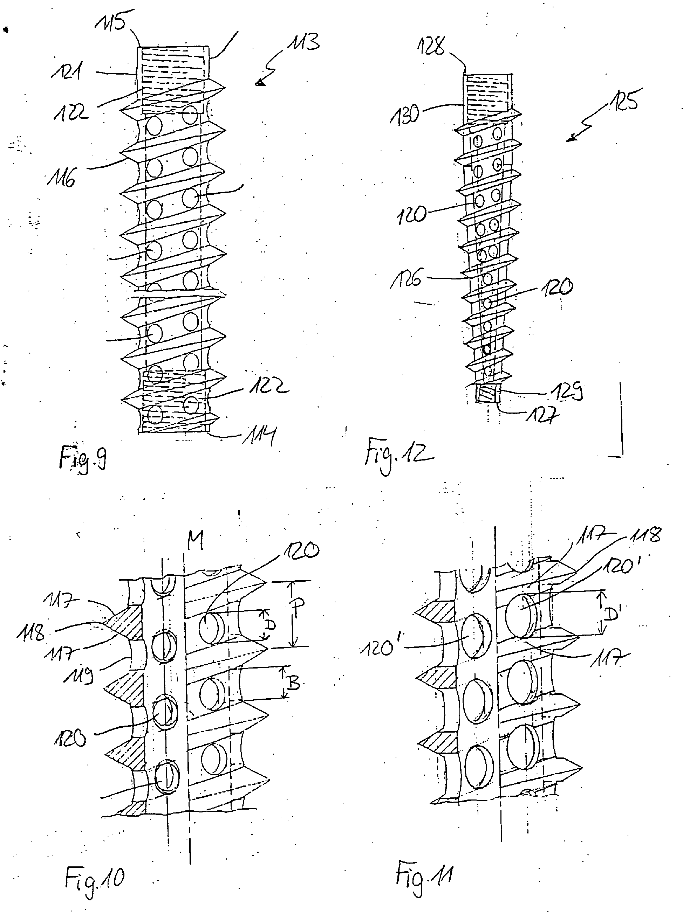

Für derartige Anwendungen ist eine in den Figuren 9 bis 11 gezeigte Abwandlung des rohrförmigen Schraubenschafts vorteilhaft. Der rohrförmige Schraubenschaft 113 besteht aus einem zylindrischen Rohr mit einem ersten Ende 114 und einem diesem gegenüberliegenden zweiten Ende 115. Das Rohr weist wie schon bei den Beispielen der Figuren 1 bis 8 an seiner Außenwand einen Knochengewindeabschnitt 116 mit einem Knochengewinde zum Einschrauben in den Knochen auf. Das Knochengewinde ist als selbstschneidendes Gewinde ausgebildet und hat in bekannter Weise Gewindeflanken 117, eine Wendelspitze 118, einen Gewindegrund 119 mit einer Breite B und eine Gewindesteigung P. In wenigstens dem Knochengewindeabschnitt 116 weist die Wandung des rohrförmigen Schafts eine Mehrzahl von Ausnehmungen 120 auf mit kreisförmigem Querschnitt auf. Die Ausnehmungen 120 sind so angeordnet, daß ihre Mitte jeweils im Gewindegrund 119 liegt und der Durchmesser D einer jeden Ausnehmung 120 ist kleiner, als die Gewindesteigung P und insbesondere nicht größer als die Breite B des Gewindegrunds, so daß in dem in den Fig. 9 und 10 dargestellten Beispiel die Ausnehmungen 120 vollständig im Gewindegrund 119 liegen und sich nicht in die Flanken 117 erstrecken. Es sind in jedem Gewindegang im Gewindegrund 119 eine Mehrzahl von auf der Schraubenlinie gleichmäßig beabstandeten Ausnehmungen 120 vorgesehen, so daß sich in axialer Richtung gesehen jeweils die Ausnehmungen eines Gewindegangs über den Ausnehmungen des darunterliegenden Gewindegangs befinden.

Wie insbesondere aus Fig. 9 ersichtlich ist, weist der rohrförmige Schaft 113 angrenzend an das erste Ende 115 einen knochengewindefreien Abschnitt 121 mit einer glatten Außenwand auf, in der keine Ausnehmungen gebildet sind. Ferner ist bei dem gezeigten Beispiel angrenzend an das erste Ende 114 und angrenzend an das zweite Ende 115 des Rohrs ein Innengewindeabschnitt 122 gebildet, der zum Verbinden mit Spitze bzw. Schraubenkopf und Aufnahmeteil dient, wie anhand der vorhergehenden Ausführungsformen beschrieben wurde.

Das in Fig. 11 gezeigte weitere abgewandelte Beispiel unterscheidet sich von der in den Figuren 9 und 10 dargestellten Beispiel dadurch, daß der Durchmesser D' der Ausnehmung 120' größer ist, als die Breite B des Gewindegrunds 119, so daß sich die Ausnehmungen 120' in die Flanken 117 des Knochengewindes hineinerstrecken, ohne jedoch die Wendelspitze 118 zu durchbrechen. Dadurch ist es möglich, die Ausnehmungen größer zu bilden, um eine bessere Fusionswirkung mit dem Knochen zu erzielen, eine Entstehung von Zähnen mit Fräswirkung beim Einschrauben wird jedoch verhindert, da die Schneidspitze des Gewindes unversehrt bleibt.

In einer weiteren, nicht dargestellten Abwandlung sind alle oder ein Teil der Ausnehmungen 120, 120' an der Außenseite der Wandung mit einer Ansenkung versehen, die eine Oberflächenrauhigkeit bildet, welche ein Ein- bzw. Anwachsen erleichtert. Der Durchmesser dieser Ansenkung in Schraubenachsrichtung ist jedoch kleiner als die Gewindesteigung P, so daß die Wendelspitze 118 unversehrt ist.

In einer weiteren Abwandlung sind die Ausnehmungen oval oder rautenförmig. Entscheidend ist, daß sie im Gewindegrund angeordnet sind und daß ihre Abmessungen so sind, daß die Schneidspitze des Knochengewindes nicht verletzt wird. Es müssen ferner nicht in jedem Gewindegang Ausnehmungen vorgesehen sein.

In einer weiteren Abwandlung erstreckt sich der Knochengewindeabschnitt 116 über die gesamte Länge des Schafts 113. Das Innengewinde 122 kann sich ebenfalls über die gesamte Länge erstrecken. Alternativ kann das Innengewinde 122 auch nur an einem Ende in einem Abschnitt oder gar nicht vorgesehen sein. Die Verbindung mit den weiteren teilen des Verankerungselement erfolgt im Fall, daß kein Innengewinde vorgesehen ist, z.B. über Paßsitz.

In einer in Fig. 12 gezeigten weiteren abgewandelten Beispiel des Schafts ist der rohrförmige Schraubenschaft 125 nicht insgesamt zylindrisch ausgebildet, sondern weist einen konischen Knochengewindeabschnitt 126 auf, der sich in Richtung zu dem mit der Spitze zu verbindenden Ende 127 verjüngt. Angrenzend an den konischen Knochengewindeabschnitt 126 erstreckt sich beidseitig jeweils bis zu den gegenüberliegenden Enden 127, 128 jeweils ein zylindrischer Abschnitt 129, 130 mit einem Innengewinde zum Verbinden mit der Spitze an einem Ende bzw. mit einem Kopf oder einem Aufnahmeteil, wie zuvor beschrieben, am anderen Ende.

In einer Abwandlung ist der mit der Spitze zu verbindende zylindrische Abschnitt 129 nicht vorgesehen, sondern das freie Ende des konischen Knochengewindeabschnitts 126 wirkt selbst als Spitze.

In den in Fig. 12 gezeigten Beispiel sind die Ausnehmungen 120 wie bei der in den Figuren 9 bis 11 gezeigten Beispiel am Gewindegrund vorgesehen. In einer weiteren Abwandlung ist der in den Figuren 1 bis 8 gezeigte rohrförmige Gewindeschaft 13 ebenfalls wenigstens in einem Abschnitt konisch ausgebildet.

## Patentansprüche

1. Verankerungselement mit einer einen Schaft (13) mit einem Knochengewindeabschnitt und einen Kopf (14; 140) aufweisenden Schraube (12) und einem Aufnahmeteil (1) zum Verbinden der Schraube mit einem stabförmigen Element (40),
wobei die Schraube und das Aufnahmeteil monoaxial miteinander verbunden sind,
wobei der Schaft (13) der Schraube rohrförmig ausgebildet ist und seine Wandung eine Mehrzahl von Ausnehmungen (18) aufweist,
**dadurch gekennzeichnet, daß** das Aufnahmeteil (70, 70') so ausgebildet ist, daß das stabförmige Element (40, 40') in dem Aufnahmeteil zur Schraubenachse versetzt gehalten ist.

2. Verankerungselement nach Anspruch 1, **dadurch gekennzeichnet, daß** der Schaft (13) und der Kopf (14; 140) als getrennte Teile ausgebildet sind.

3. Verankerungselement nach Anspruch 2, **dadurch gekennzeichnet, daß** der Schaft (13) an seinem dem Kopf zugewandten Ende (16) einen Innengewindeabschnitt und der Kopf (140) einen Abschnitt mit einem entsprechenden Außengewinde (143) aufweist derart, daß der Kopf in den Schaft einschraubbar ist.

4. Verankerungselement nach Anspruch 2, **dadurch gekennzeichnet, daß** der Kopf (14) an seinem dem Schaft zugewandten Ende eine Ausnehmung (24) aufweist, in der der Schaft (13) gehalten ist.

5. Verankerungselement nach Anspruch 4, **dadurch gekennzeichnet, daß** der Kopf (14) auf seiner dem Schaft (13) zugewandten Seite einen federnd nachgebenden Rand (25, 26) aufweist.

6. Verankerungselement nach Anspruch 4 oder 5, **dadurch gekennzeichnet, daß** der Schaft (13) angrenzend an sein dem Kopf (14) zugewandten Ende einen knochengewindefreien Abschnitt (20) aufweist.

7. Verankerungselement nach Anspruch 6, **dadurch gekennzeichnet, daß** der knochengewindefreie Abschnitt (20) im wesentlichen eine glatte Außenfläche aufweist.

8. Verankerungselement nach Anspruch 6, **dadurch gekennzeichnet, daß** der knochengewindefreie Abschnitt (20) ein metrisches Außengewinde (28') aufweist, welches mit einem entsprechenden Innengewinde (28) an der Wandung der Ausnehmung (24) zusammenwirkt.

9. Verankerungselement nach Anspruch 6, **dadurch gekennzeichnet, daß** der knochengewindefreie Abschnitt (20) eine gewellte Außenfläche (27') aufweist, die mit einer gewellten Wandung (27) der Ausnehmung (24) zusammenwirkt.

10. Verankerungselement nach einem der Ansprüche 1 bis 9, **dadurch gekennzeichnet, daß** der Kopf einen durchgehenden Kanal aufweist.

11. Verankerungselement nach einem der Ansprüche 1 bis 10, **dadurch gekennzeichnet, daß** die Spitze der Schraube ein separates Element ist, das in einen Innengewindeabschnitt des Schafts einschraubbar ist.

12. Verankerungselement nach einem der Ansprüche 1 bis 11, **dadurch gekennzeichnet, daß** der Kopf der Schraube und das Aufnahmeteil (90) einstückig ausgebildet sind, wodurch eine monoaxiale Verbindung besteht.

13. Verankerungselement nach einem der Ansprüche 1 bis 12, **dadurch gekennzeichnet, daß** ein Teil der Ausnehmungen (18) in das Knochengewinde derart hineinragt, daß die Wendelspitze durchbrochen ist.

14. Verankerungselement nach einem der Ansprüche 1 bis 12, **dadurch gekennzeichnet, daß** die Ausnehmungen (120, 120') im Gewindegrund (119) des Knochengewindes angeordnet sind und so bemessen sind, daß die Wendelspitze (118) des Knochengewindes unversehrt ist.

15. Verankerungselement nach einem der Ansprüche 1 bis 14, **dadurch gekennzeichnet, daß** der rohrförmige Schaft einen konischen Knochengewindeabschnitt (126) aufweist.

16. Verankerungselement nach einem der Ansprüche 1 bis 15, bei dem der Schaft ein rohrförmiges Schaftelement ist, mit einem Knochengewindeabschnitt (13) und mit in dem Knochengewindeabschnitt vorgesehenen Ausnehmungen (18) in der Wandung, welches an einem Ende (17) zum Aufnehmen einer Spitze ausgebildet ist und welches am gegenüberliegenden Ende (16) ein Mittel (21) zu Ineingriffbringen mit einem Schraubendreher zum Einschrauben des Schaftelements in den Knochen aufweist.

17. Verankerungselement nach Anspruch 16, **dadurch gekennzeichnet, dass** das Mittel zum Ineingriffbringen mit einem Schraubendreher stirnseitige Schlitze (21) sind.

## Claims

1. Anchoring element comprising a screw (12) having a shank (13) with a bone thread portion and a head (14; 140), and comprising a receiving part (1) for connecting the screw with a rod-shaped element (40), the screw and the receiving part being monoaxially connected to one another, and wherein the shank (13) of the screw is of tubular design and its wall has a plurality of recesses (18),
**characterized in that** the receiving part (70, 70') is designed in such a manner that the rod-shaped element (40, 40') is held offset with respect to the screw axis.

2. Anchoring element according to claim 1, wherein the shank (13) and the head (14; 140) are designed as separate parts.

3. Anchoring element according to claim 2, wherein the shank (13) comprises an inner thread portion at its end (16) directed toward the head, and the head (140) has a portion with a corresponding outer thread (143) so that the head can be screwed into the shank.

4. Anchoring element according to claim 2, wherein the head (14), at its end directed toward the shank, has a recess (24) in which the shank (13) is held.

5. Anchoring element according to claim 4, wherein the head (14) has a resiliently yielding edge (25, 26) on its side directed toward the shank (13).

6. Anchoring element according to claim 4 or 5, wherein the shank (13), adjacent to its end directed toward the head (14) has a bone thread-free portion (20).

7. Anchoring element according to claim 6, wherein the bone thread-free portion (20) substantially has a smooth surface.

8. Anchoring element according to claim 6, wherein the bone thread-free portion (20) has a metric outer thread (28') which cooperates with a corresponding inner thread (28) on the wall of the recess (24).

9. Anchoring element according to claim 6, wherein the bone thread-free portion (20) has a corrugated outer surface (27') which cooperates with a corrugated wall (27) of the recess (24).

10. Bone anchoring element according to one of claims 1 to 9, wherein the head has a channel extending through it.

11. Anchoring element according to one of claims 1 to 10, wherein the tip of the screw is a separate element which can be screwed into an inner thread portion of the shank.

12. Anchoring element according to one of claims 1 to 11, wherein the head of the screw and the receiving part (90) are designed as one piece, whereby a monoaxial connection is established.

13. Anchoring element according to one of claims 1 to 12, wherein some of the recesses (18) protrude into the bone thread in such a way that the helix crest is interrupted.

14. Anchoring element according to one of claims 1 to 12, wherein the recesses (120, 120') are arranged in the thread root (119) of the bone thread and are so dimensioned that the helix crest (118) of the bone thread is left intact.

15. Anchoring element according to one of claims 1 to 14, wherein the tubular shank has a conical bone thread portion (126).

16. Anchoring element according to one of claims 1 to 15, wherein the shank is a tubular shank element with a bone thread portion (13) and with recesses (18) in the wall in the bone thread portion, which is at one end (17) formed so as to receive a tip and which comprises at the opposite end (16) means (21) for engagement with a screw driver for screwing in the shank element into the bone.

17. Anchoring element according to claim 16, wherein the means for engagement with a screw driver are slits (21) on the front face.

## Revendications

1. Élément d'ancrage avec une vis (12), présentant une tige (13), avec un tronçon à filetage à os et une tête (14, 140), et une partie de logement (1) pour relier la vis à un élément (40) en forme de barre,
la vis et la partie de logement étant reliées ensemble de manière monoaxiale,
la tige (13) de la vis est en forme de tube et sa paroi présente une pluralité d'évidements (18),
**caractérisé en ce que** la partie de logement (70, 70') est réalisée de manière que l'élément (40, 40') en forme de barre soit maintenu dans la partie de logement, de façon décalée par rapport à l'axe de vis.

2. Élément d'ancrage selon la revendication 1, **caractérisé en ce que** la tige (13) et la tête (14; 140) sont réalisés sous forme de parties séparées.

3. Élément d'ancrage selon la revendication 2, **caractérisé en ce que** la tige (13), sur son extrémité (16) tournée vers la tête, présente un tronçon à filetage intérieur, et la tête (140) présente un tronçon ayant un filetage extérieur (143) correspondant, de manière que la tête puisse être glissée dans la tige.

4. Élément d'ancrage selon la revendication 2, **caractérisé en ce que** la tête (14) présente, sur son extrémité tournée vers la tête, un évidement (24) dans lequel la tige (13) est maintenue.

5. Élément d'ancrage selon la revendication 4, **caractérisé en ce que** la tête (14), sur son côté tourné vers la tige (13), présente un bord (25, 26) déformable élastiquement.

6. Élément d'ancrage selon la revendication 4 ou 5, **caractérisé en ce que** la tige (13) présente, en limite à son extrémité tournée vers la tête (14), un tronçon (20) exempt de filetage à os.

7. Élément d'ancrage selon la revendication 6, **caractérisé en ce que** le tronçon (20) exempt de filetage à os présente essentiellement une surface extérieure lisse.

8. Élément d'ancrage selon la revendication 6, **caractérisé en ce que** le tronçon (20) exempt de filetage à os présente un filetage extérieur (28') métrique, coopérant avec un filetage intérieur (28) correspondant, sur la paroi de l'évidement (24).

9. Élément d'ancrage selon la revendication 6, **caractérisé en ce que** le tronçon (20) exempt de filetage à os présente une surface extérieure (27') ondulée, coopérant avec une paroi (27) ondulée de l'évidement.

10. Élément d'ancrage selon l'une des revendications 1 à 9, **caractérisé en ce que** la tête présente un canal continu.

11. Élément d'ancrage selon l'une des revendications 1 à 10, **caractérisé en ce que** la pointe de la vis est un élément séparé, pouvant être vissé dans un tronçon de filetage intérieur de la tige.

12. Élément d'ancrage selon l'une des revendications 1 à 11, **caractérisé en ce que** la tête de la vis et la partie de logement (90) sont réalisées d'une seule pièce, donnant une liaison monoaxiale.

13. Élément d'ancrage selon l'une des revendications 1 à 12, **caractérisé en ce qu'**une partie des évidements (18) pénètre dans le filetage à os, de manière que la pointe d'hélice soit interrompue.

14. Élément d'ancrage selon l'une des revendications 1 à 12, **caractérisé en ce** les évidements (120, (120') sont disposées dans le fond de filetage (119) du filetage à os et dimensionnées de manière que la pointe d'hélice (118) du filetage à os soit intouchée.

15. Élément d'ancrage selon l'une des revendications 1 à 14, **caractérisé en ce** a tige en forme de tube présente un tronçon de filetage à os (126) conique.

16. Élément d'ancrage selon l'une des revendications 1 à 15, dans lequel la tige est un élément de tige en forme de tube avec un tronçon de filetage à os (13), et avec des évidements (18) prévus dans le tronçon de filetage à os, dans la paroi, élément qui, à une extrémité (17), est réalisé pour recevoir une pointe et qui, à l'extrémité (16) opposée, présente un moyen (21) de mise en prise avec un tournevis, pour visser l'élément de tige dans l'os.

17. Élément d'ancrage selon la revendications 16, **caractérisé en ce** le moyen de mise en prise avec un tournevis est formé par des fentes (21) situées côté frontal.
